# EUROPEAN PATENT APPLICATION

(11) **EP 4 785 795 A1**
(43) Date of publication of application: **05.08.2026**
(21) Application number: 24872493.2
(22) Date of filing: 27.09.2024
(51) Int. Cl.: A01N 63/20, A01N 43/60, A01P 3/00, A61K 31/495, A61K 35/74, A61P 31/04, A61P 39/02, C07D 241/08, C12N 1/20, C12P 13/00

(54) **AFLATOXIN PRODUCTION-INHIBITING BACTERIUM, AFLATOXIN PRODUCTION INHIBITOR PRODUCED BY AFLATOXIN PRODUCTION-INHIBITING BACTERIUM AND METHOD FOR PRODUCING SAME, AND METHOD FOR CONTROLLING AFLATOXIN CONTAMINATION USING SAID BACTERIUM OR SAID INHIBITOR**

(30) Priority: 28.09.2023 JP 2023167713
(71) Applicant: Teikyo University, Tokyo 173-8605 (JP)
(72) Inventor: SAKUDA Shohei, Utsunomiya-shi, Tochigi 320-8551 (JP); UCHIDA Kenichi, Utsunomiya-shi, Tochigi 320-8551 (JP); HAKOSHIMA Noriko, Utsunomiya-shi, Tochigi 320-8551 (JP); ISHIJIMA Natsumi, Utsunomiya-shi, Tochigi 320-8551 (JP); SAKODA Ayaka, Utsunomiya-shi, Tochigi 320-8551 (JP); SUNAOKA Masaki, Utsunomiya-shi, Tochigi 320-8551 (JP); TERADA Maho, Utsunomiya-shi, Tochigi 320-8551 (JP)
(74) Representative: Cohausz & Florack
(86) International application number: PCT/JP2024/034612
(87) International publication number: WO 2025/070711

(57) **Abstract**

Provided are a novel strain of an aflatoxin production inhibiting bacterium which is a novel microorganism having a high aflatoxin production inhibiting activity; a method for efficiently producing an inhibitor that includes an aflatoxin production inhibitor produced by an aflatoxin production inhibiting bacterium including the novel strain and a chemical substance of which the effect is improved compared with the aflatoxin production inhibitor; and a method for controlling aflatoxin contamination using the aflatoxin production inhibiting bacterium or the aflatoxin production inhibitor. The aflatoxin production inhibitor comprises a culture product of Klebsiella sp. or Raoultella sp. or a compound represented by general formula (1) 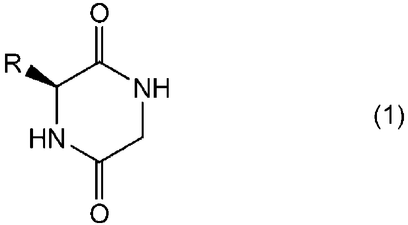 wherein R represents a linear or branched C1-4 alkyl group.

## Description

### TECHNICAL FIELD

The present invention relates to a novel strain of an aflatoxin production inhibiting bacterium, which is a novel microorganism; an aflatoxin production inhibitor produced by an aflatoxin production inhibiting bacterium including the novel strain, and a method of producing the same; and a method of controlling aflatoxin contamination using the same.

### BACKGROUND ART

Among toxic compounds called mycotoxins, which are secondary metabolites of fungi, aflatoxin is the most serious problem.

Microorganisms that inhibit aflatoxin production have been identified, including microorganisms that suppress aflatoxin production by inhibiting the growth of an aflatoxin producing microorganism; microorganisms that can suppress aflatoxin production without inhibiting growth; microorganisms having aflatoxin-degrading activity; and microorganisms that adsorb aflatoxin to cells. For example, it has been found that bacteria such as Bacillus subtilis, Nannocystis exedens, Bacillus pumilus, Pseudomonas syringae, Ralstonia paucula, and Burkholderia cepacia have an effect of suppressing aflatoxin production along with the growth of an aflatoxin producing microorganism. It has also been found that Streptococcus lactis suppresses aflatoxin production without affecting the growth of an aflatoxin producing microorganism, and that Achromobacter xylosoxidans inhibits norsolorinic acid production by a norsolorinic-acid-producing strain, norsolorinic acid being a biosynthetic precursor of aflatoxin. Further, yeasts of the genera Kluyveromyces, Candida, and Pichia have been reported to inhibit aflatoxin production. Studies have also been conducted to search for microorganisms that degrade aflatoxin, and it has been shown that Flavobacterium aurantiacum, Mycobacterium fluoranthenivorans, Rhodococcus erythropolis, Myxococcus fulvus, and the like degrade aflatoxin. Further, lactic acid bacteria such as Lactobacillus rhamnosus have been identified as bacteria that adsorb aflatoxin to cells (Non-Patent Literature 1).

Non-Patent Literature 2 reports that Klebsiella sp. and Klebsiella pneumonia, and a culture supernatant thereof, degrade aflatoxin.

Further, several diketopiperazines are known as compounds that inhibit aflatoxin production. For example, Non-Patent Literature 3 describes cyclo (L-Leu-L-Pro) produced by Achromobacter xylosoxidans, and Non-Patent Literature 4 describes cyclo (L-Ala-L-Pro) and cyclo (L-Val-L-Pro) produced by Stenotrophomonas sp.

### CITATION LIST

### NON-PATENT LITERATURE

[Non-Patent Literature 1] Sakuda, Shohei, "Prevention of aflatoxin contamination by biocontrol," Mycotoxins, Vol. 63, No. 2, pp. 217-224 (2013).
[Non-Patent Literature 2] Ning, M., Zhang, S., Xie, Y., Wang, W., Gao, Y.: Aflatoxin B1 removal by three bacterial strains and optimization of fermentation process parameters. Biotechnology and Applied Biochemistry, 66(6), 930-938 (2019)
[Non-Patent Literature 3] Yan, P.S., Song, Y., Sakuno, E., Nakajima, H., Nakagawa, H., Yabe, K.: Cyclo (L-Leucyl-L-Prolyl) produced by Achromobacter xylosoxidans inhibits aflatoxin production by Aspergillus parasiticus. Appl Environ Microbiol, 70, 7466-7473 (2004)
[Non-Patent Literature 4] Jermnak, U., Chinaphuti, A., Poapolathep, A., Kawai, R., Nagasawa, H., Sakuda, S.: Prevention of aflatoxin contamination by a soil bacterium of Stenotrophomonas sp. that produces aflatoxin production inhibitors. Microbiology, 159, 902-912 (2013)

### SUMMARY OF INVENTION

### TECHNICAL PROBLEM

An object of the present invention is to provide a novel strain of an aflatoxin production inhibiting bacterium, which is a novel microorganism having high aflatoxin production inhibiting activity; an aflatoxin production inhibitor produced by an aflatoxin production inhibiting bacterium including the novel strain; an efficient method for producing an inhibitor containing a highly effective agent; and a method of controlling aflatoxin contamination using the aflatoxin production inhibiting bacterium or the aflatoxin production inhibitor.

### SOLUTION TO PROBLEM

[1] An aflatoxin production inhibitor comprising a culture product of Klebsiella species (Klebsiella sp.) or Raoultella species (Raoultella sp.).
[2] An aflatoxin production inhibitor comprising a compound represented by the following general formula (1): 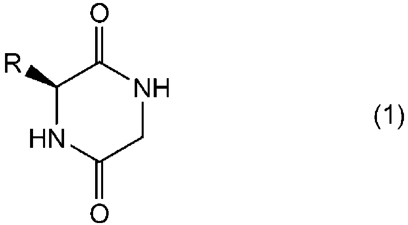 wherein R is a linear or branched alkyl group having 1 to 4 carbon atoms.
[3] A method of producing an aflatoxin production inhibitor, the method comprising:
   culturing Klebsiella species (Klebsiella sp.) or Raoultella species (Raoultella sp).
[4] The method according to claim 3, further comprising:
   purifying, from the culture product obtained in the culturing, a compound represented by the following general formula (1): 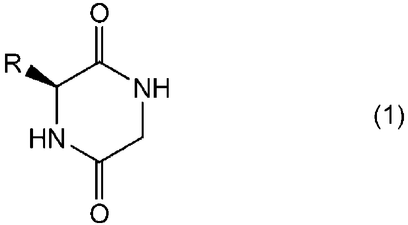
   wherein R is a linear or branched alkyl group having 1 to 4 carbon atoms.
[5] A method of inhibiting aflatoxin production comprising:
   using the aflatoxin production inhibitor according to claim 1 or 2.
[6] A method of controlling aflatoxin contamination, comprising:
   using the aflatoxin production inhibitor according to claim 1 or 2 to inhibit aflatoxin production by an aflatoxin producing microorganism.
[7] A compound represented by the following general formula (1):
   wherein R is a methyl group. 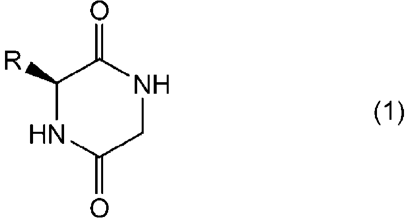
[8] Klebsiella aerogenes having Accession No. NITE BP-03899.

### ADVANTAGEOUS EFFECTS OF INVENTION

According to the present invention, it is possible to provide a novel strain of an aflatoxin production inhibiting bacterium, which is a more effective novel microorganism; an aflatoxin production inhibitor having higher activity and produced by an aflatoxin production inhibiting bacterium including the novel strain; efficient methods of producing the same; and a method of controlling aflatoxin contamination using the aflatoxin production inhibiting bacterium or the aflatoxin production inhibitor.

### BRIEF DESCRIPTION OF THE DRAWINGS

[Fig. 1] FIG. 1 is a graph showing results of analyzing an aflatoxin concentration in peanuts after peanuts immersed in a liquid obtained by tenfold serial dilution of culture broth of Klebsiella aerogenes strain KTTM were inoculated with an aflatoxin producing microorganism and cultured.
[Fig. 2] FIG. 2 is a graph showing results of analyzing an aflatoxin concentration in peanuts after peanuts immersed in a liquid obtained by tenfold serial dilution of culture broth of various microorganisms belonging to the genus Klebsiella or the genus Raoultella were inoculated with an aflatoxin producing microorganism and cultured.
[Fig. 3] FIG. 3 is a drawing showing structural formulae of various diketopiperazines evaluated for aflatoxin production inhibiting activity, and concentrations (IC₅₀) of respective evaluation samples that reduce an amount of aflatoxin B₁ to 1/2.

### DESCRIPTION OF EMBODIMENTS

### (Novel Strain of Aflatoxin Production Inhibiting Bacterium)

The novel aflatoxin production inhibiting bacterium of the present invention is Klebsiella aerogenes strain KTTM (Accession No. NITE BP-03899). A culture product of the aflatoxin production inhibiting bacterium (e.g., culture broth, culture supernatant, cultured cells, or a lysate of the cultured cells, or an extract of any of these culture products) has aflatoxin production inhibiting activity. Therefore, the bacterium can be used as a producing microorganism for an aflatoxin production inhibitor.

The bacteriological characteristics of the KTTM strain are as follows.

On nutrient agar, colony morphology is cream-colored, circular, lenticular, entire, smooth, opaque, and buttery. Based on 16S rDNA base-sequence analysis results, the strain is identified as Klebsiella aerogenes. The strain is a motile Gram-negative rod, is catalase-positive, is oxidase-negative, and ferments glucose and produces gas. The strain exhibits activities such as β-galactosidase, lysine decarboxylase, and ornithine decarboxylase, does not exhibit activities such as arginine dihydrolase, urease, and gelatinase, and oxidizes various sugars. The strain can grow at 37°C and 45°C.

### (Aflatoxin Production Inhibiting Bacterium)

In the present invention, for example, for inhibiting aflatoxin production, for controlling aflatoxin contamination, or for producing an aflatoxin production inhibitor, microorganisms belonging to the genus Klebsiella (Klebsiella species) and microorganisms belonging to the genus Raoultella (Raoultella species) can be used in addition to the KTTM strain. Examples of microorganisms belonging to the genus Klebsiella include, for example, Klebsiella aerogenes, Klebsiella pneumoniae, and Klebsiella oxytoca. Examples of microorganisms belonging to the genus Raoultella include, for example, Raoultella planticola, Raoultella terrigena, and Raoultella ornithinolytica.

### (Aflatoxin Production Inhibitor)

The aflatoxin production inhibitor of the present invention comprises at least a culture product of Klebsiella species or Raoultella species, and/or a compound represented by the following general formula (1): 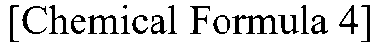 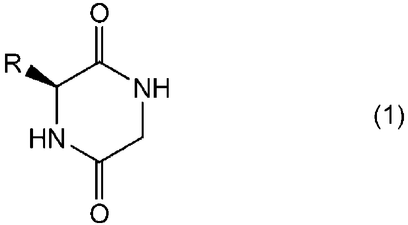 wherein R is a linear or branched alkyl group having 1 to 4 carbon atoms, and may further comprise other components.

The aflatoxin production inhibitor of the present invention may (i) comprise solely a culture product of Klebsiella species or Raoultella species, (ii) comprise solely one or more compounds represented by general formula (1), or (iii) comprise, in combination, a culture product of Klebsiella species or Raoultella species and one or more compounds represented by general formula (1).

There is no particular limitation on a method of producing the aflatoxin production inhibitor, and the method can be appropriately selected depending on the intended purpose; however, the aflatoxin production inhibitor is preferably produced by the method of producing an aflatoxin production inhibitor of the present invention described below.

The culture product in the aflatoxin production inhibitor of the present invention is not particularly limited as long as it is a culture product of Klebsiella species or Raoultella species, and can be appropriately selected depending on the intended purpose; examples include culture broth, culture supernatant, cultured cells, and a lysate of the cultured cells.

The culture product may also be an extract thereof. Examples of an extract of the culture product include, for example, an extract supernatant obtained by adding an appropriate organic solvent (e.g., ethanol, methanol, or acetone) to the culture product to form a suspension, and then performing centrifugation, membrane filtration, or the like to separate the suspension from cells, and a product obtained by subjecting the extract supernatant to a commonly used isolation and purification treatment.

These may be used alone or in combination of two or more. Among these, culture broth, culture supernatant, or cultured cells are preferable.

The Klebsiella species or Raoultella species may also be one that has been subjected to treatment such as irradiation or other mutagenesis treatment so as to enhance aflatoxin production inhibiting activity.

The content of the culture product in the aflatoxin production inhibitor is not particularly limited, and can be appropriately selected depending on the intended purpose. The aflatoxin production inhibitor may be the culture product itself.

The compound represented by general formula (1) in the aflatoxin production inhibitor of the present invention is not particularly limited as long as R is a linear or branched alkyl group having 1 to 4 carbon atoms; examples include cyclo (L-Ala-Gly) in which R is a methyl group, cyclo (L-Abu(2)-Gly) in which R is an ethyl group, cyclo (L-Val-Gly) in which R is an i-propyl group, cyclo (L-Nva-Gly) in which R is an n-propyl group, cyclo (L-NorLeu-Gly) in which R is an n-butyl group, cyclo (L-Leu-Gly) in which R is an i-butyl group, cyclo (L-Ile-Gly) in which R is a sec-butyl group, and cyclo (L-tert-Leu-Gly) in which R is a tert-butyl group.

As R, a methyl group, an ethyl group, an i-propyl group, an n-propyl group, or an i-butyl group is more preferable; a methyl group, an ethyl group, an i-propyl group, or an n-propyl group is more preferable; an ethyl group, an i-propyl group, or an n-propyl group is further preferable; an ethyl group or an i-propyl group is further preferable; and an ethyl group is particularly preferable.

The content of the compound represented by general formula (1) in the aflatoxin production inhibitor is not particularly limited, and can be appropriately selected depending on the intended purpose. The aflatoxin production inhibitor may be the compound represented by general formula (1) itself.

The compound represented by general formula (1) can be appropriately selected depending on the intended purpose, and may be produced by the method of producing an aflatoxin production inhibitor of the present invention described below, which includes a culturing step and a purification step, or may be produced by chemical synthesis.

The method of producing the compound by chemical synthesis is not particularly limited, and can be appropriately selected from known methods depending on the intended purpose; examples include the method described in Thajudeen, H.; Park, K.; Moon, S. S.; Hong, I. S. An efficient green synthesis of proline-based cyclic dipeptides under water-mediated catalyst-free conditions. Tetrahedron Lett. 2010, 51, 1303-1305.

Specifically, one methyl ester of an amino acid constituting a compound represented by general formula (1) (e.g., L-Ala-OCH₃ and Boc-Gly are coupled using a dehydrating agent to obtain a dipeptide protected with a Boc group (tert-butoxycarbonyl group) and a methyl ester group, and then these protecting groups are deprotected and cyclized, whereby a compound represented by general formula (1) (e.g., cyclo (L-Ala-Gly)) can be synthesized.

Other components in the aflatoxin production inhibitor are not particularly limited as long as they do not impair the effects of the present invention, and examples include optional pesticide components and pesticide adjuvants.

The content of such other components in the aflatoxin production inhibitor is not particularly limited, and can be appropriately selected depending on the intended purpose.

The pesticide component is not particularly limited and can be appropriately selected depending on the intended purpose, examples include active ingredients of pesticides listed below.

Examples of the pesticides include fungicides, bactericides, antiviral agents, plant resistance inducers, insecticides, acaricides, nematicides, insect growth regulators, insect attractants, herbicides, plant growth regulators, synergists, phytotoxicity-reducing agents, bird repellents, fertilizers, and soil conditioners. These may be used alone or in combination of two or more.

The pesticide adjuvant contains a carrier, a surfactant, and other adjuvants, and may further contain other components.

The carrier is not particularly limited as long as it can be used for agricultural and horticultural applications, and can be appropriately selected depending on the intended purpose; examples include liquid carriers and solid carriers. These may be used alone or in combination of two or more.

Examples of the liquid carrier include water; alcohols such as isopropyl alcohol and ethylene glycol; ketones such as cyclohexanone and methyl ethyl ketone; ethers such as propylene glycol monomethyl ether and diethylene glycol mono-n-butyl ether; aliphatic hydrocarbons such as kerosene and light oil; aromatic hydrocarbons such as xylene, trimethylbenzene, tetramethylbenzene, methylnaphthalene, and solvent naphtha; amides such as N-methyl-2-pyrrolidone; esters such as glycerin esters of fatty acids; and vegetable oils such as soybean oil and rapeseed oil.

Examples of the solid carrier include animal- and plant-derived powders such as starch, activated carbon, soybean flour, wheat flour, wood flour, fish meal, and powdered milk; and mineral powders such as talc, kaolin, bentonite, zeolite, diatomaceous earth, white carbon, clay, alumina, calcium carbonate, potassium chloride, and ammonium sulfate.

The surfactant is not particularly limited and can be appropriately selected depending on the intended purpose; examples include nonionic surfactants, anionic surfactants, cationic surfactants, and amphoteric surfactants. These surfactants may be used alone or in combination of two or more.

Examples of the nonionic surfactants include polyoxyethylene alkyl ethers, polyoxyethylene alkylaryl ethers, polyoxyethylene styrylphenyl ethers, polyoxyethylene alkyl esters, polyoxyethylene sorbitan alkylates, polyoxyethylene phenyl ether polymers, polyoxyethylene alkylene arylphenyl ethers, polyoxyethylene alkylene glycols, and polyoxyethylene polyoxypropylene block polymers.

Examples of the anionic surfactants include ligninsulfonates, alkylarylsulfonates, dialkyl sulfosuccinates, polyoxyethylene alkylaryl ether sulfates, alkylnaphthalenesulfonates, and polyoxyethylene styrylphenyl ether sulfates.

Examples of the cationic surfactants include alkylamine salts.

Examples of the amphoteric surfactants include quaternary ammonium salt alkylbetaines and amine oxides.

Other adjuvants are not particularly limited and can be appropriately selected depending on the intended purpose; examples include binders, thickeners, fixatives, preservatives, antifungal agents, solvents, stabilizers for pesticide active ingredients, antioxidants, UV absorbers, crystal precipitation inhibitors, antifoaming agents, physical-property improvers, and colorants.

The binders, thickeners, and fixatives are not particularly limited and can be appropriately selected depending on the intended purpose; examples include dextrin, cellulose, methylcellulose, ethylcellulose, carboxymethylcellulose, hydroxyethylcellulose, hydroxypropylcellulose, hydroxypropyl methylcellulose, carboxymethyl starch, pullulan, sodium alginate, ammonium alginate, propylene glycol alginate, guar gum, locust bean gum, gum arabic, xanthan gum, gelatin, casein, polyvinyl alcohol, polyethylene oxide, polyethylene glycol, ethylene-propylene block polymers, sodium polyacrylate, and polyvinylpyrrolidone.

The dosage form of the aflatoxin production inhibitor is not particularly limited and can be appropriately selected depending on the intended purpose; examples include emulsifiable concentrates, suspensions, wettable powders, water-soluble formulations, liquid formulations, sol formulations (flowable formulations), water-dispersible granules, dusts, fine granules, granules, tablets, oil formulations, spray formulations, fumigant formulations, aerosol formulations, and paste formulations. Among these, liquid formulations are preferable.

The method of producing each formulation is not particularly limited, and the formulations can be produced by known methods.

The aflatoxin production inhibitor may also be mixed or used in combination with other antimicrobial agents (e.g., fungicides, bactericides, antiviral agents, and plant resistance inducers), insecticides, acaricides, nematicides, insect growth regulators, insect attractants, herbicides, plant growth regulators, synergists, phytotoxicity-reducing agents, bird repellents, fertilizers, soil conditioners, and the like.

### (Method of Producing Aflatoxin Production Inhibitor)

Among the methods of producing an aflatoxin production inhibitor of the present invention, a method of producing an aflatoxin production inhibitor comprising a culture product of Klebsiella species (preferably the KTTM strain) comprises at least a step of culturing Klebsiella species or Raoultella species (culturing step), and may further comprise other steps.

Further, among the methods of producing an aflatoxin production inhibitor of the present invention, a method of producing an aflatoxin production inhibitor comprising a compound represented by general formula (1) (particularly cyclo (L-Ala-Gly)) comprises at least a step of culturing Klebsiella species (preferably the KTTM strain) or Raoultella species (culturing step) and a step of purifying, from the culture product obtained in the culturing step, the compound represented by general formula (1) (purification step), and may further comprise other steps.

The culturing in the culturing step is performed by inoculating Klebsiella species (preferably the KTTM strain) or Raoultella species into a nutrient medium (hereinafter also simply referred to as "medium") and culturing the microorganism at a temperature suitable for production of the aflatoxin production inhibitor.

The nutrient medium is not particularly limited and can be appropriately selected depending on the intended purpose; for example, known media conventionally used for culturing Klebsiella species (preferably Klebsiella aerogenes) or Raoultella species can be used.

Nutrient sources to be added to the nutrient medium are not particularly limited and can be appropriately selected depending on the intended purpose; examples include nitrogen sources such as commercially available peptone, yeast extract, meat extract, corn steep liquor, cottonseed meal, peanut meal, soybean meal, NZ-amine, casein, sodium nitrate, ammonium nitrate, and ammonium sulfate; carbon sources such as tomato paste, glycerin, sucrose, starch, glucose, galactose, mannose, dextrin, molasses, and fats; and inorganic salts such as sodium chloride, phosphates, calcium carbonate, and magnesium sulfate.

In addition to such nutrient sources, trace metal salts and animal oils, vegetable oils, mineral oils, and the like as antifoaming agents may also be added as components in the medium.

These materials may be any materials that can be utilized by Klebsiella species (preferably the KTTM strain) or Raoultella species and are useful for production of the aflatoxin production inhibitor, and all known culture materials can be used.

A seed culture for production of the aflatoxin production inhibitor is not particularly limited and can be appropriately selected depending on the intended purpose; examples include agar-grown cells of Klebsiella species (preferably the KTTM strain) or Raoultella species.

Culture conditions are not particularly limited and can be appropriately selected depending on the intended purpose; however, aerobic culture conditions are preferable.

The culturing method may be any of solid (agar) culture such as slant culture and plate culture, or liquid culture; however, liquid culture is preferable from the viewpoint of producing the aflatoxin production inhibitor in a large amount. The liquid culture may be shaking culture, stationary culture, or stirring culture; however, shaking culture is preferable, and rotary shaking culture is more preferable. In the case of large-scale culture, culture may be performed using a fermenter or the like.

The culture temperature is not particularly limited as long as it is within a range in which growth of Klebsiella species (preferably the KTTM strain) or Raoultella species is not substantially inhibited and the aflatoxin production inhibitor can be produced, and can be appropriately selected depending on the producing microorganism to be used; however, 20°C to 37°C is preferable.

The culture period is not particularly limited and can be appropriately selected in accordance with accumulation of the aflatoxin production inhibitor. Typically, accumulation of the aflatoxin production inhibitor reaches a maximum after 3 to 7 days of culture.

The purification step is a step of purifying the compound represented by general formula (1) from the culture product obtained in the culturing step. For the culture product, the description regarding the culture product in the aflatoxin production inhibitor of the present invention can be applied as it is.

The method of purifying the compound represented by general formula (1) from the culture product is not particularly limited and can be appropriately selected depending on the intended purpose; examples include solvent extraction, partition or adsorption chromatography, high-performance liquid chromatography, and reversed-phase chromatography. These methods may be used in combination.

The purification step may be performed once; however, performing the purification step multiple times is preferable in that the yield of the compound represented by general formula (1) is improved.

The method of confirming that a substance obtained in the purification step is the compound represented by general formula (1) is not particularly limited and can be appropriately selected depending on the intended purpose; examples include electrospray ionization time-of-flight mass spectrometry (ESI-TOFMS), a method of measuring optical rotation using a polarimeter, proton nuclear magnetic resonance spectroscopy (¹H-NMR), and carbon nuclear magnetic resonance spectroscopy (¹³C-NMR).

### (Method of Controlling Aflatoxin Contamination)

The method of controlling aflatoxin contamination of the present invention is a method of inhibiting aflatoxin production by an aflatoxin producing microorganism using the aflatoxin production inhibitor of the present invention. The method is not particularly limited as long as it is a method of applying the aflatoxin production inhibitor to a target object to which the aflatoxin producing microorganism has adhered and/or infected, or a target object for which occurrence of adhesion and/or infection of the aflatoxin producing microorganism is desired to be prevented, and can be appropriately selected depending on the intended purpose. In the method of controlling aflatoxin contamination of the present invention, without being limited thereto, aflatoxin contamination can be controlled by inhibiting aflatoxin production by the aflatoxin producing microorganism.

The target object is not particularly limited and can be appropriately selected depending on the intended purpose; examples include plants and agricultural crops.

Examples of the agricultural crops include cereals such as corn, rice, buckwheat, and Job's tears; nuts such as peanuts, pistachio nuts, and Brazil nuts; spices such as nutmeg, chili pepper, and paprika; beans such as coffee beans; sesame; cottonseed; and the like.

The method of applying the aflatoxin production inhibitor is not particularly limited and can be appropriately selected depending on the intended purpose; examples include a method of broadcast application of the aflatoxin production inhibitor as it is or the aflatoxin production inhibitor diluted with water or the like (e.g., spraying, misting, atomizing, dusting, granule application, water-surface application, or box application), a method of soil application (e.g., mixing-in or drench irrigation), a method of surface application (e.g., coating, seed dressing, or covering), and a method of immersion.

The application amount of the aflatoxin production inhibitor is not particularly limited, and can be appropriately selected depending on various conditions such as the concentration of active ingredients in the aflatoxin production inhibitor, the formulation form, the target disease and the type of crop, the degree of damage caused by the disease, the application site, the application method, the application timing, the type and amount of chemicals and fertilizers to be mixed or used in combination, and weather.

The application concentration of the aflatoxin production inhibitor is not particularly limited, and can be appropriately selected depending on the intended purpose.

### EXAMPLES

Hereinafter, the present invention will be described in detail by way of Examples; however, these Examples do not limit the scope of the present invention.

### <Example 1: Culturing of Aflatoxin Production Inhibiting Bacterium>

In this Example, culture broth of Klebsiella aerogenes strain KTTM was prepared.

A loopful of a colony of the KTTM strain grown on a Bennett solid medium (agar 1.5%, glucose 1%, peptone 0.2%, meat extract 0.1%, yeast extract 0.1%, pH 7.2) was inoculated into 7 mL of a Bennett liquid medium (glucose 1%, peptone 0.2%, meat extract 0.1%, yeast extract 0.1%, pH 7.2) in a test tube, and the culture was shake-cultured at 27.5°C for 2 days (150 rpm). To the resulting culture broth, an 80% aqueous glycerol solution was added to adjust the glycerol concentration to 20%, and the culture was stored at -80°C.

Then, 0.21 mL of the 20% glycerol stock solution of the KTTM strain was inoculated into 7 mL of the Bennett liquid medium in a test tube, and shake-cultured at 27.5°C for 3 days (150 rpm).

### <Test Example 1: Evaluation of Aflatoxin Production Inhibiting Activity (1)>

### (1) Preparation of spore suspension

As an aflatoxin B₁-producing microorganism, Aspergillus flavus IMF47798 (obtained from the Reference Strain of the International Union of Microbiological Societies) was cultured on a plate of potato dextrose agar medium (PDA medium; Difco, MD, USA) at 27°C for 14 days. Spores were scraped from the mycelium with a loop and suspended in distilled water containing 0.1 wt% Tween 20 to prepare a spore suspension at 1.1×10^5 CFU/µL.

### (2) Evaluation of aflatoxin production inhibiting activity

Ten milliliters of distilled water was added to a beaker (100 mL capacity) containing 100 dried peanuts (approximately 0.5 g each; Chiba Handachi peanuts; Midorikawa Shoten, Chiba) from which shells and testa had been removed, and the peanuts were autoclaved at 120°C for 20 minutes. The autoclaved peanuts were immersed in serially diluted solutions of the culture broth prepared in Example 1 using sterilized Millipore water, and then transferred to a 24-well microplate at one peanut per well.

As a control, peanuts were immersed in sterilized Millipore water instead of the diluted solution of the culture broth of Example 1 and then transferred to a 24-well microplate at one peanut per well.

Each well was inoculated with 5 µL/well of the spore suspension prepared in Test Example 1(1), and statically cultured at 25°C for 14 days.

Each mold-grown peanut was transferred to a Falcon tube (15 mL), 5.0 mL of a 90 vol% aqueous acetonitrile solution was added, and the peanut was crushed with a spatula for extraction. Two milliliters of the resulting crushed extract was applied to an aflatoxin purification column (Autoprep MF-A 1000, Resonac, Tokyo), 1.0 mL of the column effluent was collected into a Falcon tube (15 mL), and freeze-dried. After freeze-drying, the residue was dissolved in 0.5 mL of a 10 vol% aqueous acetonitrile solution, filtered through a filter (Minisart RC4, Sartorius, Gottingen, Germany), and the mass of aflatoxin B₁ was measured by high-performance liquid chromatography under the measurement conditions described below.

### [HPLC measurement conditions]

### -HPLC-

- Apparatus: SHIMADZU 20A HPLC system (pump: LC20AD; autosampler: SIL-20AC; oven: CTO-20AC; fluorescence detector: RF-20A xs)
- Column: Capcell-pak C₁₈ column UG120 (4.6 mm i.d., 250 mm length; Osaka Soda)
- Elution: acetonitrile:methanol:water (1:3:6, v/v/v), isocratic elution
- Flow rate: 1.0 mL/min
- Retention time: aflatoxin B₁ (12.5 min)
- Detection: fluorescence at 450 nm (excitation wavelength: 365 nm)

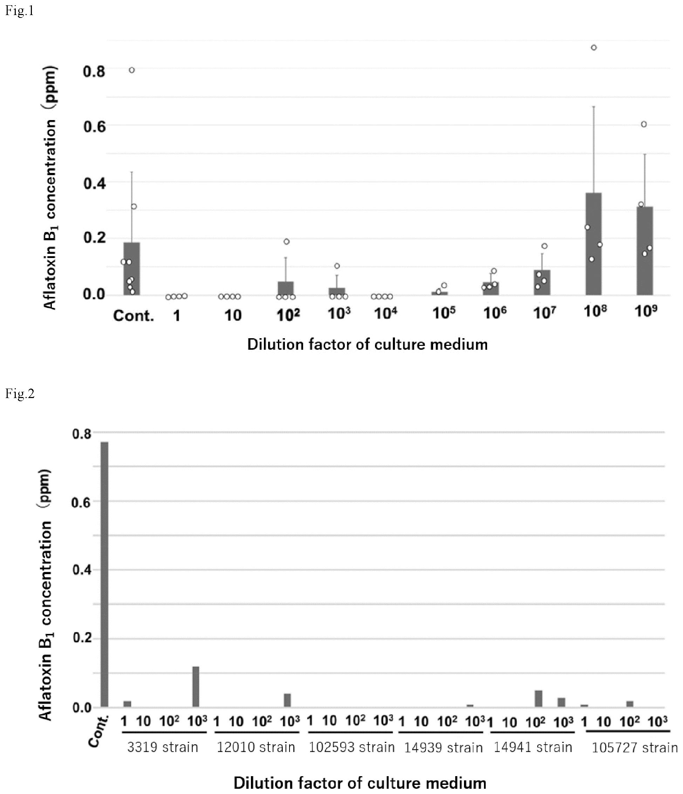

The results are shown in FIG. 1. After peanuts immersed in tenfold serial dilutions of the culture broth of the KTTM strain (cell number: 2.3×10^9 cells/mL) were inoculated with an aflatoxin producing microorganism and cultured, the aflatoxin concentration in the peanuts was analyzed. As a result, aflatoxin production was strongly suppressed compared with the control (immersed in water) even with a 100,000-fold dilution (dilution factor: 10^5). In the figure, "∘" (open circles) indicates the amount of aflatoxin in each individual peanut; however, the experiment exhibits large variation in aflatoxin production among individuals due to differences in peanut shape and the like.

It was separately confirmed that the culture broth of Klebsiella aerogenes does not degrade aflatoxin.

### <Test Example 2: Evaluation of Aflatoxin Production Inhibiting Activity (2)>

Culturing of Klebsiella pneumoniae NBRC 3319, Klebsiella aerogenes NBRC 12010, Klebsiella oxytoca NBRC 102593, Raoultella planticola NBRC 14939, Raoultella terrigena NBRC 14941, and Raoultella ornithinolytica NBRC 105727 was performed in the same manner as in Example 1.

Evaluation of aflatoxin production inhibiting activity was performed in the same manner as in Test Example 1.

The results are shown in FIG. 2. After peanuts immersed in tenfold serial dilutions (up to 1,000-fold) of culture broth of each of Klebsiella pneumoniae NBRC 3319, Klebsiella aerogenes NBRC 12010, Klebsiella oxytoca NBRC 102593, Raoultella planticola NBRC 14939, Raoultella terrigena NBRC 14941, and Raoultella ornithinolytica NBRC 105727 were inoculated with an aflatoxin producing microorganism and cultured, the aflatoxin concentration in the peanuts was analyzed. As a result, for all six strains, aflatoxin production was strongly suppressed compared with the control (immersed in water) even with a 1,000-fold dilution (dilution factor: 10^3). The control represents an average value for 12 peanuts, and the others represent an average value for 2 peanuts.

In this Test Example as well, Klebsiella aerogenes (NBRC 12010 strain), which is the same species as the KTTM strain evaluated in Test Example 1, was used; however, the KTTM strain exhibited stronger aflatoxin production inhibiting activity than the NBRC 12010 strain.

### <Example 2: Production of Aflatoxin Production Inhibitor (2)>

In this Example, an active substance, cyclo (L-Ala-Gly), was purified from culture broth of Klebsiella aerogenes strain KTTM, and an aflatoxin production inhibitor comprising the active substance was prepared.

### (1) Culturing step

A pre-culture broth was obtained by inoculating 0.21 mL of the 20% glycerol stock solution of the KTTM strain prepared in Example 1 into 7 mL of the Bennett liquid medium in a test tube and shake-culturing at 27.5°C for 2 days (150 rpm). In the main culture, 3 mL of the pre-culture broth was inoculated into 100 mL of the Bennett liquid medium in a 500 mL Erlenmeyer flask and shake-cultured at 27.5°C for 5 days (150 rpm).

### (2) Purification step

A culture supernatant obtained by removing cells from 3 L of culture broth obtained in the culturing step by centrifugation (5,000×g, 10 min) was passed through an activated charcoal column packed using water as a solvent (Activated Charcoal, 100 g, FUJIFILM Wako Chemicals). The column was then washed with 300 mL of water, followed by elution with 500 mL of 10 vol% ethanol. A concentrate (18.5 mL) obtained by concentrating the resulting 10 vol% ethanol eluate fraction was subjected to three runs of reversed-phase high-performance liquid chromatography (RP-HPLC) under the measurement conditions described below to purify the active substance.

### [RP-HPLC measurement conditions: 1st run]

- Column: Capcellpak C₁₈ column (250 mm length, 10 mm i.d.; Osaka Soda)
- Eluents: A, 0.1 vol% aqueous trifluoroacetic acid / B, 100 vol% acetonitrile
- Elution conditions: linear gradient elution from A:B=100:0 to A:B=0:100 over 15 minutes
- Flow rate: 2.5 mL/min
- Detection: UV 300 nm
- Yield of active fraction: 40.6 mg

### [RP-HPLC measurement conditions: 2nd run]

- Column: Capcellpak C₁₈ column (250 mm length, 4.6 mm i.d.; Osaka Soda)
- Eluents: A, 0.1 vol% aqueous trifluoroacetic acid / B, 100 vol% acetonitrile
- Elution conditions: linear gradient elution from A:B=100:0 to A:B=0:100 over 15 minutes
- Flow rate: 1.0 mL/min
- Detection: UV 220 nm
- Yield of active fraction: 4.0 mg

### [RP-HPLC measurement conditions: 3rd run]

- Column: Senshu Pak DOCOSIL SP-100 column (250 mm length, 4.6 mm i.d.; Senshu Scientific)
- Elution: isocratic elution (aqueous solution containing 10 mmol/L ammonium acetate)
- Flow rate: 1.0 mL/min
- Detection: UV 210 nm
- Retention time of active substance: 5.2 min
- Yield of active substance: 1.15 mg

### (3) Identification step

The obtained active substance was identified as cyclo (L-Ala-Gly) based on the retention time in the RP-HPLC described above and comparison of the following physicochemical properties with those of a standard.

(a) By electrospray ionization time-of-flight mass spectrometry (ESI-TOFMS; positive ion mode), the observed value was m/z 129.0666 (M+H)⁺ (calcd for C₅H₉N₂O₂, 129.0664).
(b) The specific optical rotation was [α]²⁷_{D} = -13.8 (c = 0.06, H₂O).
(c) By nuclear magnetic resonance spectroscopy, the ¹H and ¹³C NMR spectra measured at 25°C in deuterated dimethyl sulfoxide (DMSO-d₆) at 500 MHz were as follows:
   δ_{H} (DMSO-d₆, 500 MHz): 8.16 (1H, br.s, NH), 7.97 (1H, br.s, NH), 3.84(1H, q, J = 7 Hz), 2.72 (2H, m), 1.25 (3H, d, J = 7 Hz); δ_{C} (DMSO-d₆, 125 MHz): 168.9, 166.3, 49.7, 44.5, 18.7.

### <Test Example 3: Evaluation of Aflatoxin Production Inhibiting Activity (2)>

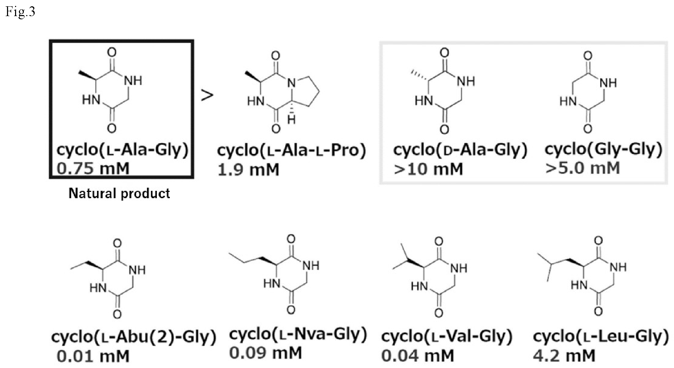

As evaluation samples, cyclo (L-Ala-Gly) shown in FIG. 3 and related compounds thereof were used.

As cyclo (L-Ala-Gly), a commercially available product (Bachem, Budendorf, Switerland) was used.

As cyclo (L-Ala-L-Pro), a compound synthesized in Non-Patent Literature 2 was used.

Cyclo (D-Ala-Gly), cyclo (L-Abu(2)-Gly), cyclo (Gly-Gly), cyclo (L-Val-Gly), cyclo (L-Nva-Gly), and cyclo (L-Leu-Gly) were synthesized and used by the method described below.

D-Ala-OMe-HCl (0.415 g, 3 mmol; Watanabe Chemical Industries, Hiroshima, Japan) was dissolved in 3 mL of dry DMF. Boc-Gly-OH (0.526 g, 3 mmol; Watanabe Chemical Industries), 2-(1H-benzotriazol-1-yl)-1,1,3,3-tetramethyluronium hexafluorophosphate (HBTU) (1.37 g, 3.6 mmol), and N,N-diisopropylethylamine (DIPEA) (0.38 g, 6.0 mmol) were added to the solution, and the mixture was stirred overnight at room temperature. Ethyl acetate (100 mL) was added to the reaction solution, and the organic layer was washed with 5% NaHCO₃ (120 mL) and 5% NaCl (120 mL) and dried over anhydrous Na₂SO₄. After evaporation, the residue (0.451 g) was subjected to chromatography using a silica gel column (Silica gel 60 (0.063-0.200 mm), 20 g; Merck, Darmstadt, Germany), and a Boc- and OMe-protected dipeptide (0.296 g) was obtained by isocratic elution with n-hexane-EtOAc (1:1). This protected dipeptide (0.296 g) was deprotected and cyclized by autoclaving in water (30 mL) at 121°C for 4 hours. This aqueous solution was evaporated, and the residue (0.238 g) was purified by silica gel column chromatography (Silica gel 60 (0.063-0.200 mm), 20 g; CH₂Cl₂:MeOH (9:1)) to obtain cyclo (D-Ala-Gly) (42 mg).

Similarly, 84 mg of cyclo (L-Abu(2)-Gly), 22 mg of cyclo (Gly-Gly), 106 mg of cyclo (L-Val-Gly), 66 mg of cyclo (L-Nva-Gly), and 137 mg of cyclo (L-Leu-Gly) were obtained from, respectively, 6 mmol of Gly-OMe-HCl and Boc-L-Abu(2)-OH, Gly-OMe-HCl and Boc-Gly-OH, L-Val-OMe-HCl and Boc-Gly-OH, L-Nva-OMe-HCl and Boc-Gly-OH, and L-Leu-OMe-HCl and Boc-Gly-OH (Watanabe Chemical Industries), in the same manner.

Cyclo(D-Ala-Gly): ESI-Q/TOFMS m/z 129.0666 (M+H)⁺ (calcd for C₅H₉N₂O₂, 129.0664); [α]²⁷_{D} +3.7 (c = 0.26, H₂O); δ_{H} (DMSO-d₆, 500 MHz): 8.16 (1H, br.s, NH), 7.98 (1H, br.s, NH), 3.84 (1H, q, J = 7 Hz), 3.72 (2H, m), 1.25 (3H, d, J = 7 Hz); δ_{C} (DMSO-d₆, 125 MHz): 168.6, 166.3, 49.7, 44.5, 18.7.

Cyclo(L-Abu(2)-Gly): ESI-Q/TOFMS m/z 143.0823 (M+H)⁺ (calcd for C₆H₁₁N₂O₂, 143.0821); [α]²⁷_{D} +21.2 (c = 0.64, H₂O); δ_{H} (DMSO-d₆, 500 MHz): 8.17 (1H, br.s, NH), 8.00 (1H, br.s, NH), 3.77 (1H, d, J = 17 Hz), 3.72 (1H, m), 3.68 (1H, d, J = 17 Hz), 1.61-1.77 (2H, m), 0.85 (3H, t, J = 7 Hz); δ_{C} (DMSO-d₆, 125 MHz): 167.8, 166.1, 55.1, 44.3, 25.9, 8.8.

Cyclo(Gly-Gly): ESI-Q/TOFMS m/z 115.0506 (M+H)⁺ (calcd for C₄H₇N₂O₂, 115.0508); δ_{H} (DMSO-d₆, 500 MHz): 8.02 (1H, br.s, NH), 3.70 (2H); δ_{C} (DMSO-d₆, 125 MHz): 166.1, 44.3.

### (Evaluation Method)

A sample solution (100 µL) was added to 1.9 mL of potato dextrose liquid medium in each well of a microplate (24 wells). A spore suspension of Aspergillus flavus (5 µL) was inoculated into the medium, and statically cultured at 25°C for 4 days. A mixture of 100 µL of culture broth and 400 µL of water:acetonitrile (9:1, v/v) was filtered (Minisart RC4, Sartorius, Gottingen, Germany), and the amount of aflatoxin B₁ in the filtrate was analyzed by HPLC using a Capcell pak C₁₈ UG 120 column (250 mm×4.6 mm i.d.; Osaka Soda). The HPLC was performed under conditions of isocratic elution using acetonitrile:methanol:water (1:3:6, v/v/v), 20 minutes, a flow rate of 1.0 mL, and fluorescence detection at 450 nm (excitation wavelength: 365 nm).

The results are shown in FIG. 3. The concentrations shown in FIG. 3 are concentrations (IC₅₀) of the respective evaluation samples that reduce the amount of aflatoxin B₁ to 1/2.

The diketopiperazines represented by the following formula, which can be used as an active ingredient of the aflatoxin production inhibitor of the present invention: 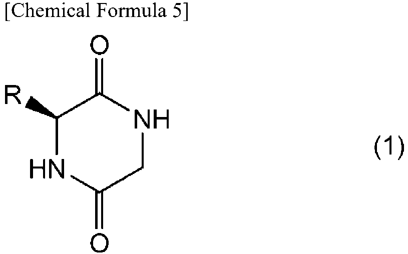 wherein R is a linear or branched alkyl group having 1 to 4 carbon atoms, exhibited higher aflatoxin production inhibiting effects than cyclo (L-Ala-L-Pro), which is known to have aflatoxin production inhibiting activity. In particular, cyclo (L-Abu(2)-Gly), in which the methyl group of the natural product cyclo (L-Ala-Gly) was substituted with an ethyl group, showed extremely excellent aflatoxin production inhibiting activity, with activity increased 75-fold relative to the natural product.

It should be noted that cyclo (D-Ala-Gly), which is a stereoisomer of cyclo (L-Ala-Gly), and cyclo (Gly-Gly), which does not have substituent R (i.e., the group corresponding to R is a hydrogen atom), did not exhibit sufficient aflatoxin production inhibiting activity.

### INDUSTRIAL APPLICABILITY

The aflatoxin production inhibitor of the present invention has excellent aflatoxin production inhibiting activity. Therefore, it can be used in a method of controlling aflatoxin contamination.

### ACCESSION NUMBER

Klebsiella aerogenes strain KTTM was internationally deposited on June 8, 2023, at the National Institute of Technology and Evaluation (NITE) Patent Microorganisms Depositary (NPMD) (Room 122, 2-5-8 Kazusa-Kamatari, Kisarazu-shi, Chiba 292-0818, Japan) as Accession No. NITE BP-03899.

## Claims

1. An aflatoxin production inhibitor comprising a culture product of Klebsiella species (Klebsiella sp.) or Raoultella species (Raoultella sp.).

2. An aflatoxin production inhibitor comprising a compound represented by the following general formula (1): 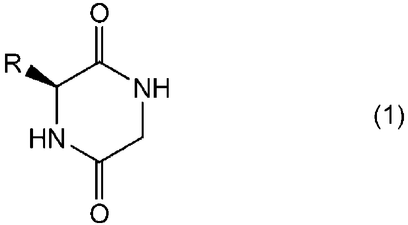 wherein R is a linear or branched alkyl group having 1 to 4 carbon atoms.

3. A method of producing an aflatoxin production inhibitor, the method comprising:
culturing Klebsiella species (Klebsiella sp.) or Raoultella species (Raoultella sp).

4. The method according to claim 3, further comprising:
purifying, from the culture product obtained in the culturing, a compound represented by the following general formula (1): 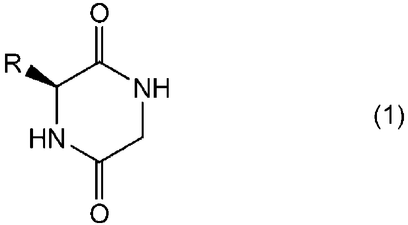
wherein R is a linear or branched alkyl group having 1 to 4 carbon atoms.

5. A method of inhibiting aflatoxin production comprising:
using the aflatoxin production inhibitor according to claim 1 or 2.

6. A method of controlling aflatoxin contamination, comprising:
using the aflatoxin production inhibitor according to claim 1 or 2 to inhibit aflatoxin production by an aflatoxin producing microorganism.

7. A compound represented by the following general formula (1):
wherein R is a methyl group.

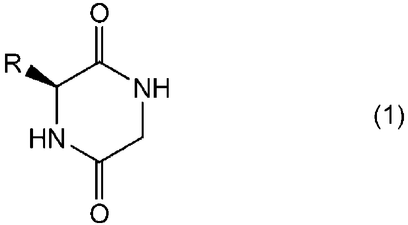

8. Klebsiella aerogenes having Accession No. NITE BP-03899.
